# EUROPEAN PATENT SPECIFICATION

(11) **EP 1 505 089 B1**
(45) Date of publication and mention of the grant of the patent: **11.01.2006**
(21) Application number: 03077473.1
(22) Date of filing: 07.08.2003
(51) Int. Cl.: C08F 20/60, C12N 15/87, A61K 47/48

(54) **Cationic (alkyl)acrylamide derivative, polymer based on these derivates, and transfection system comprising said polymer**
Kationisches (Alkyl)acrylamid, hiervon abgeleitetes Polymeres sowie dieses enthaltendes Transfektions-System
(Alkyl) acrylamide cationique, polymere base sur lequelle ainsique systeme de transfection synthetique le contenant

(43) Date of publication of application: 09.02.2005
(73) Proprietor: OctoPlus Technologies B.V., 2333 CL Leiden (NL)
(72) Inventor: Funhoff, Arjen Mike, 3561 LR Utrecht (NL); van Nostrum, Cornelis Franciscus, 5251 CV Vlijmen (NL); Hennink, Wilhelmus Everhardus, 2743 CZ Waddinxveen (NL)
(74) Representative: Prins, Adrianus Willem

(56) References cited:
- EP-A- 0 463 508
- EP-A- 1 022 340
- WO-A-97/15680
- VAN DE WETERING P ET AL: "2-(dimethylamino)ethyl methacrylate based (co)polymers as gene transfer agents" JOURNAL OF CONTROLLED RELEASE, ELSEVIER SCIENCE PUBLISHERS B.V. AMSTERDAM, NL, vol. 53, no. 1-3, 30 April 1998 (1998-04-30), pages 145-153, XP004121265 ISSN: 0168-3659

## Description

The present invention is in the field of synthetic transfection or blocking systems useful in the delivery of gene constructs, RNA or DNA fragments to cells, especially to cells in living organisms. More in particular, the invention relates to cationic polymers which are suitable to be used as a DNA RNA or gene delivery system, which can be used in, e.g. gene therapy applications.

Particularly, the present invention relates to cationic acrylamide derivatives, and cationic (alkyl)acrylamide derivatives. In addition, the invention relates to polymers on the basis said derivates as monomer. Further, the invention relates to a transfection system comprising said polymer.

Gene therapy is seen as a promising method to correct hereditary defects or to treat life threatening diseases such as cancer and AIDS. In gene therapy, nucleic acid fragments or gene constructs are brought into target cells in order to compensate for a missing gene or to introduce a new functionality in such cells. These nucleic acid fragments or gene constructs are preferably incorporated in plasmids.

If reconstructed plasmids are applied to an organism *per* se this generally leads to low expression of the introduced gene, if any. There are three main reasons for this low expression. First, the plasmids will hardly ever reach the cell population where they are intended to be incorporated, due to degradation and elimination processes. Second, if the plasmids do reach the target cells, they cannot simply pass the cellular membrane, because of the strongly polar nature and the size of the plasmids. Third, if a plasmid does invade a target cell, it normally will be enclosed in an endosome, which will convert into a lysosome. In the lysosome, the plasmid will be degraded so that the incorporated gene cannot be expressed.

For the above reasons, in gene therapy plasmids or other gene constructs are complexed with a carrier or vehicle.

In recent years, many efforts have been made in the research on potentially suitable transfection systems, both of viral and non-viral (cationic lipids and cationic polymers) origin. These transfection systems should deliver the desired gene or DNA fragment to the target cell and cause it to be expressed to a high degree.

The present invention is related to synthetic transfection systems, and particularly to transfection systems within the family of cationic polyacrylates and poly(alkyl)acrylates and the corresponding acrylamides. This family of cationic polymers is described in WO-A-97/15680. Said international patent application teaches a synthetic transfection system based on polyacrylates, polyacrylamides, poly(C₁₋₆ alkyl)acrylates or poly(C₁₋₆ alkyl)acrylamides which contain cationic substituents. In general, and more in particular, to water-soluble or water dispersible transfection systems, wherein organic cationic moieties are attached to the polyacrylates or poly(alkyl)acrylate backbone or the backbone of the corresponding acrylamides.

Preferably, the cationic groups attached to the carboxyl group are of the formula -R₃-N(R₄)(R₅), wherein R₃ represents a C₁₋₆ alkylene group or be replaced with inert substituents, and wherein R₄ and R₅, which may be the same or different, represent a hydrogen atom, a C₁₋₆ alkyl group or an aryl group. Instead of the afore-mentioned acrylate moieties, acrylamide moieties can also be present in WO-A-97/15680, preferably moieties of the formula C(O)NR₄R₅.

The cationic group comprises most preferably a dimethyl amino ethyl group. Dimethylamino ethyl groups as well as other tertiary amines, present in acryl based polymers, will at least partially be protonated under physiological conditions, yielding a cationic structure which is able to bind nucleic acid structures such as RNA and DNA electrostatically, and, in this way, condense with the nucleic acids.

The present invention provides an improvement over the known cationic transfection systems taught in WO-A-97/15680 in that it provides polymers to be used in transfection systems which polymers have a high degradation rate at physiological conditions, and especially in an aqueous environment at a temperature of 35-39°C and a pH of 6.5-8. This improvement is achieved by a particular group of cationic polyacrylamide and poly(alkyl)acrylamide derivatives.

Particularly, in a first aspect the present invention relates to a polymer based on a monomer of formula (I)

CH₂=C(R¹)-C(O)NH-CH₂-CHCH₃-O-C(O)-O-CH₂-CH₂-N(CH₃)₂ (I)

wherein R¹ is either a hydrogen atom, or a C₁₋₆-alkyl group; and preferably either a hydrogen atom or a C₁₋₃ alkyl group, and more preferably a methyl group. In a preferred embodiment, the polymer is a homopolymer.

In a second aspect, the present invention relates to the monomer as defined in the previous paragraph. In the most preferred embodiment the compound carries as substituent R¹ a methyl group; this compound is identified as HPMA-DMAE herein-below.

This monomer can very suitably be prepared by a two-step synthesis, wherein in step (i) N',N'-dimethylaminoethanol (DMAE) is activated by 1,1'-carbonyldiimidazole; which activated alcohol is in step (ii) reacted with N-(2-hydroxypropyl)-(R¹-substituted) acrylamide (HPR¹A). This process forms a further aspect of the present invention.

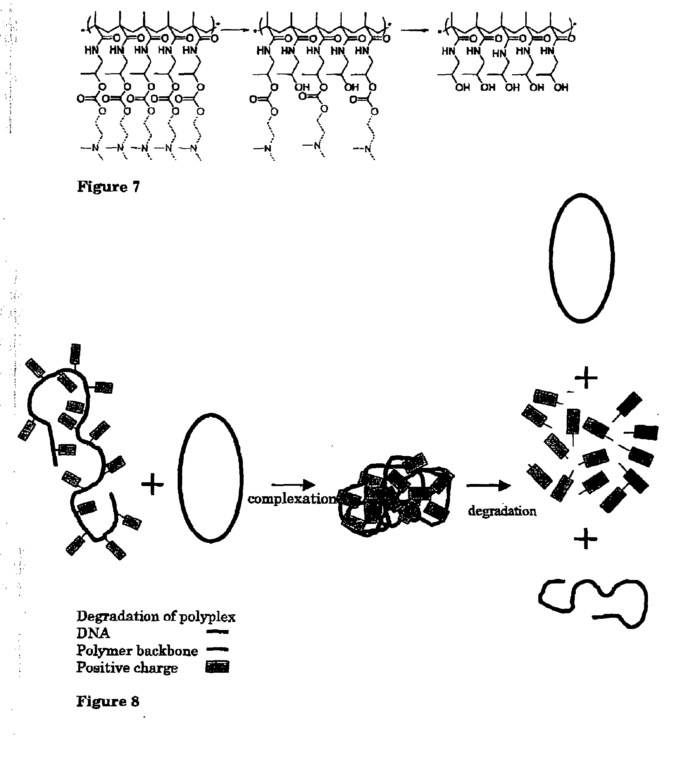

It is assumed that the advantageous degradation properties of the monomer of the present invention is attributed to the carbonyl moiety introduced between the dimethylaminoethanol starting group and the acrylamide starting compound. In Example 3, herein-below, the degradation characteristics of HPMA-DMAE are illustrated. In examples 5 and 6, the degradation of the polymer complexed with DNA is illustrated, wherein the degradation behaviour of poly(dimethyl aminoethyl methacrylate) (p(DMAEMA), one of the most preferred polymers within the scope of WO-A-97/15680, is used as a reference. Additionally, it is shown that this degradation behaviour is retained in the polymerised form. In Fig 7 the possible degradation process is shown. The degradation is based on hydrolysis of the - O-C(O)-O- bond. This hydrolysis does not or does not substantially occur at a pH of about 5 nor in a medium that does not favour hydrolysis, such as in DMSO.

For this reason, the polymer of the present invention can suitably be prepared by radical polymerization of the monomer of formula (I) and optionally other monomers (*vide infra*) in an aqueous environment having a pH of about 5.0, which pH can for instance be adjusted using HCl; or alternatively in DMSO or another polar aprotic solvent. A suitable polymerization initiator is 2,2'-azoisobutyronitrile (AIBN) when water is not used as the reaction medium. Suitable preparation techniques are described in G. Odian, Principles of Polymerization , Chapter 3 "Radical Chain Polymerization", John Wiley and Sons, Inc. New York (1991) as well as in references to which this handbook refers.

Preferably, the radical polymerization is carried out in the presence of a chain transfer reagent, e.g. β-mercapto ethanol, 2-aminoethane thiol or mercapto acetic acid. Copolymers can be obtained by mixing monomers in the desired ratios and amounts and subsequently subjecting this mixture to radical polymerization.

The polymer based on monomers of formula (I) may as part of the polymer backbone comprise other hydrophobic and hydrophilic moieties. The present invention therefore comprises both homopolymers of acrylamides and (C₁₋₆ alkyl) acrylamides and copolymers of these amides, comprising different acrylate, acrylamide, (alkyl) acrylate or (alkyl) acrylamide units, and other units such as methylmethacrylate, triethylene glycol methacrylate, and polyethylene glycol methacrylate, hydroxyethylmethacrylate, glyercylmethacrylate, laurylmethacrylate, butylmethacrylate, N-isopropylacrylamide, N-(3-dimethylamino) propyl) methacrylamide and so on. Further, it is not necessary that all backbone units represent acrylamide -like moieties. A part of the units may be formed by e.g. N-vinyl pyrrolidone or vinyl acetate.

In yet a further aspect, the present invention relates to a synthetic transfection system which comprises at least one cationic, water soluble or water dispersible polymer of the invention as a carrier, and further comprises a nucleic acid fragment, such as an RNA or a DNA fragment, for instance in the form of a plasmid, a gene construct or an oligonucleotide. Oligonucleotides can be used as blocking structures, e.g., for controlling protein synthesis, in cells.

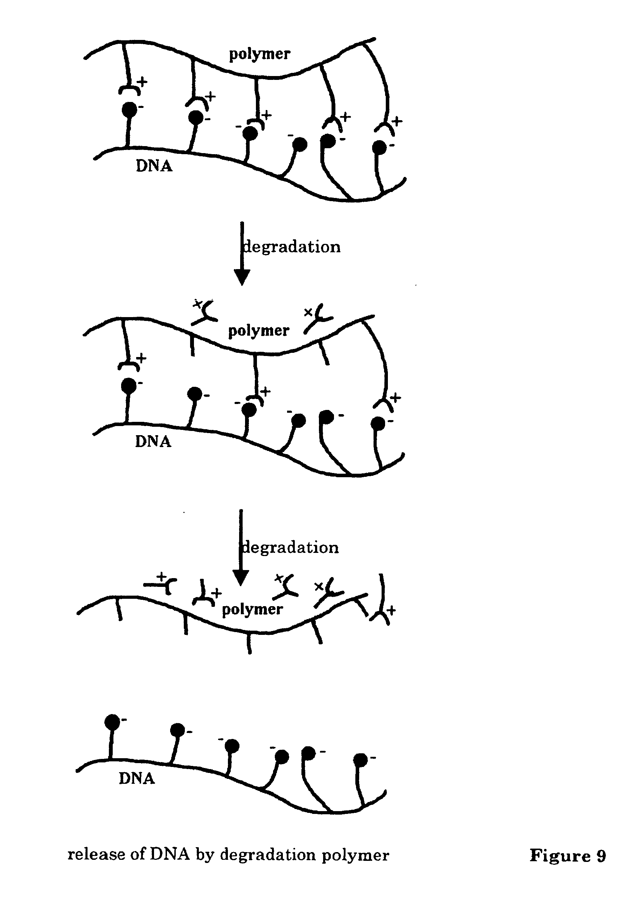

The transfection system of the present invention is formed by incubating nucleic acid molecules and polymers of the invention. This process is schematically shown in the left hand part of Fig 8; the right hand part of said Fig 8 showing the degradation of the complex under physiological conditions. Also Fig 9 shows the release of the nucleic acid from the complex in a schematic view.

More in particular, the delivery systems themselves can easily be prepared by contacting the polymers of the invention and the nucleic acid fragments, such as DNA fragments under conditions where the polymer is positively charged, preferably at a pH of 7.2 in a suitable buffer system (e.g. a PBS or HEPES buffer) at room temperature.

In a preferred embodiment the polymer-polynucleotide complexes are prepared in the presence of a viscosity increasing substance, preferably in the presence of sucrose. The addition of a viscosity increasing substance makes it possible to obtain smaller particles.

The preparation of the delivery system normally is completed within 10 minutes complexation time. The preparation step can be followed by a separation step wherein the nucleic acid-polymer complex is separated from the unbound polymer. In a subsequent step the complex comprising nucleic acid and the carrier polymer of the invention can be lyophilisized, preferably in the presence of a pharmaceutically acceptable cryoprotectant such as sucrose or manitol. In lyophilisized condition the complex can be stored for a long period of time.

The cationic moieties depicted in Fig 8 and the polymer backbone are not toxic to cells, and are excreted or otherwise removed from the system, wherein the complexes are used as transfection system.

The weight ratio of the carrier polymers to the nucleic acid fragments, and especially to the DNA or RNA fragments, seems critical. Suitable results are obtained when using weight ratios polymer to nucleic acid fragments of between 0.1 and 200; preferably between 1 and 20, most preferably between 2 and 5. The molecular weight and/or number of the polymers used can be easily adjusted to the nature of the plasmid to be transported. Normally, polymers having a molecular weight of from 1,000 to 500,000 can suitably be used as a DNA or RNA carrier. Preferably, the molecular weight of the cationic polymers used in accordance with the present invention is higher than 80.000 Da, more preferably higher than 100.000 Da, most preferably higher than 250.000 Da. The molecular weight of the polymers used in the present invention can be controlled by using and maintaining suitable reaction conditions in the polymerization process.

It is noted that the condensed particles comprising the polymer of the invention and nucleic acid fragments can be enclosed or incorporated in known drug delivery systems, *e*.*g*. in liposomes or hydrogels.

Genes to be incorporated into carrier systems or vehicles to be used in the synthetic transfection system are among others documented in
- McKusick, V.A. Mendelian inheritance in man, catalogs of autosomal dominant, autosomal recessive, and X-linked pheno-types. Eight edition. John Hopkins University Press (1988).
- Stanbury, J.B., Wyngaarden, J. B., Frederickson, D.S., Goldstein, J.L. and Brown, M.S. The metabolic basis of inherited disease. Fifth edition. Mc Graw-Hill (1983).

Vehicles to be used in embodiments of the present invention include viral and non-viral regulatory elements for expression and/or replication.

### These vehicles are well known in the field.

Suitable transfection systems are able to target a gene construct to the aimed cell population. The transfection system of the present invention therefore preferably comprises at least one group that selectively recognizes proteins associated with the surface of the target cells. Such targeting moieties or homing devices are known to the skilled person and comprise, e.g., tri and tetra antennary cluster glycosides, transferrin or other protein constructs, monoclonal antibodies against cell membrane proteins, ligands for cell surface associated receptors and binding fragments of derivatives of said targeting moieties, polyacrylamide or poly(alkyl)acrylamide system of the present invention to transported gene fragments are delivered to hepatocytes through the galactose receptor of hepatocytes. Furthermore, the presence of recognizable structures covalently or non-covalently coupled to the polymer part of a polymer-nucleic acid complex facilitates the incorporation of the nucleic acid fragment, e.g., a gene construct, in the target cell.

Moreover, the transfection system can be adapted to allow the gene construct to leave endosomes in the cellular system. Thereto membrane destabilizing structures, in particular polypeptide fragments, are conjugated to the water soluble or water dispersible cationic polymer systems of the invention. Such destabilize the endosomal membrane systems. The plasmids incorporating a gene construct so reach the cytoplasm of the target cell, where the gene construct can be expressed in the nucleus. Examples of such membrane destabilizing structures which are suitably used in accordance with the present invention are fusogenic structures, e.g., certain peptides and (parts of) viral coating proteins, for instance peptides derived from hemagglutinin protein of the influenza virus (see in this respect, *e.g.,* Plank *et al*. The influence of Endosome-Disruptive Peptides on Gene Transfer Using Synthetic Virus-Like Gene Transfer Systems, J. Biol. Chem. 269 (1994), 12918-12924), and particularly INF-7.

Other compounds useful in accordance with the present inventions are endosome destabilizing compounds such as chloroquine. It is noted that chloroquine is only used in *in vitro* applications, because it is toxic *in vivo.* Since the invention is directed to both *in* vivo and *in vitro* applications, this embodiment is within the scope of the invention.

In a further embodiment, the invention relates to a method for introducing nucleic acid fragments in target cells, comprising contacting these nucleic acid fragments with a polymer of the invention and subsequently by contacting the obtained transfection system with target cells.

The carrier system of the present invention can be used both in *in vivo* and in *in vitro* applications.

The invention is further described in the drawings, wherein:
Fig. 1 shows the degradation behaviour of HPMA-DMAE (◆) and the corresponding formation of HPMA (■) in time at 37 °C at pH = 10.0 (A), at pH = 7.4 (B) and at pH 5.0 (C).
Fig. 2 shows the degradation of p(HPMA-DMAE) in time at 37°C and at pH = 7.4;
Fig. 3 shows a number of biophysical characteristics of p(HPMA-DMAE) based polyplexes, and particularly the particle size. (Zave; Z-average diameter, determined by dynamic light scattering (DLS))(◆) and zeta potential (■) as function of the polymer to DNA ratio;
Fig. 4 shows the degradation of polyplexes based on p(HPMA-DMAE) at pH = 7.4 (▲) and at pH = 5.0 (■), as well as on the basis of p(DMAEMA) (◆); in Fig. 4(A) the particle size is monitored; in Fig. 4 (B) the signal intensity is followed in time;
Fig. 5 shows the results of gel electrophoresis of polyplexes based on p(HPMA-DMAE) at pH = 7.4 and at pH = 5.0 and of polyplexes based on p(DMAEMA) at pH = 7.4, incubated at 37 °C; lane 1: free DNA; lanes 2-7: p(DMAEMA) based polyplexes (pH = 7.4) incubation time 0, 2, 4, 7, 24 and 48 hours, respectively, lanes 8-13: p(HPMA-DMAE) based polyplexes (pH = 7.4) incubation time 0, 2, 4, 7, 24 and 48 hours, respectively; and lanes 14-19: p(HPMA-DMAE) based polyplexes (pH = 5.0) incubation time 0, 2, 4, 7, 24 and 48 hours, respectively;
Fig. 6 shows the transfection efficiency (A) and toxicity (B) of polyplexes based on p(HPMA-DMAE) without (■) and with (▲) the membrane disrupting peptide INF-7; p(DMAEMA) (◆) is used as a reference;
Fig. 7 shows the degradation of p(HPMA-DMAE) in chemical formulae;
Fig. 8 schematically shows the complexation of circular DNA by p(HPMA-DMAE) and the degradation process; and
Fig. 9 schematically shows the release of the DNA upon degradation of the polymer.

The invention will now be described on the basis of the following nonlimiting examples. In the examples, as well as in the above description all percentages are percentages by weight drawn to the total composition unless otherwise indicated.

### Example 1 Synthesis and characterisation of (p)HPMA-DMAE.

Synthesis of the monomer was performed in two steps. In the first step N'N'-dimethylaminoethanol was activated by 1,1'-carbonyldiimidazole in a good yield of 68% and good purity (>97% by NMR). In the second step the reaction between the activated alcohol and N-(2-hydroxypropyl) methacrylamide (HPMA) was followed in time until no free HPMA was detected anymore with ¹H-NMR.

### Example 2: Polymerization of HPMA-DMAE to p(HPMA-DMAE)

Radical polymerisation of the monomer formed in Example 1 was performed in an aqueous 1M HCl solution, with pH adjusted to pH 5 to prevent degradation of the monomer; and also in DMSO polymers were formed in a yield of 75% (in water) and 80% (in DMSO). The higher yield in DMSO may be explained because in DMSO degradation of the monomer is prevented to a higher extent. The weight average molecular weight was 160 kDa (in water) and 63 kDa (in DMSO), the numer average molecular weight was 20 kDa (in water) and 14 kDa (in DMSO).

### Example 3: Degradation of the monomer and polymer.

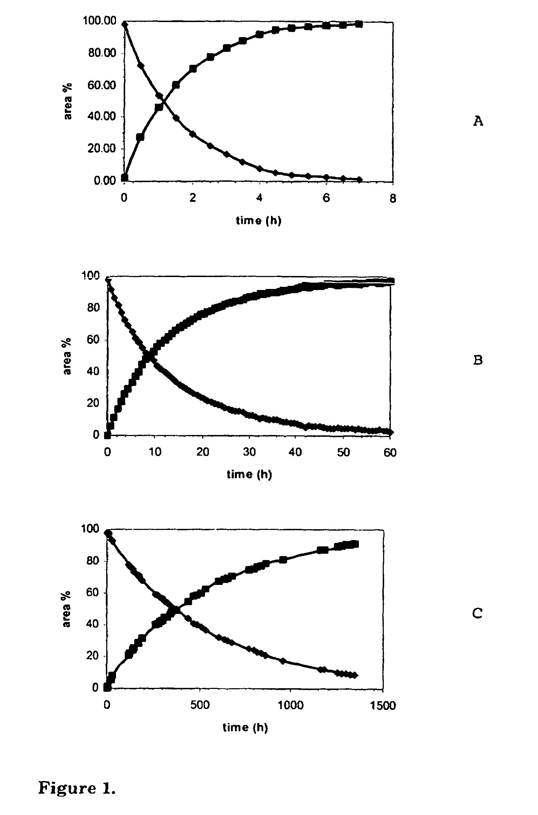

Degradation of the monomer was followed with RP-HPLC at 37 °C. The relative peak areas of the monomer and its degradation product HPMA as a function of time are depicted in figure 1(A) for pH 10, 1(B) for pH 7.4 and 1(C) for pH 5.0. From these curves the half-life of the monomer can be calculated at these pHs, being 1.1 hour, 9.6 hour and 380 hours respectively.

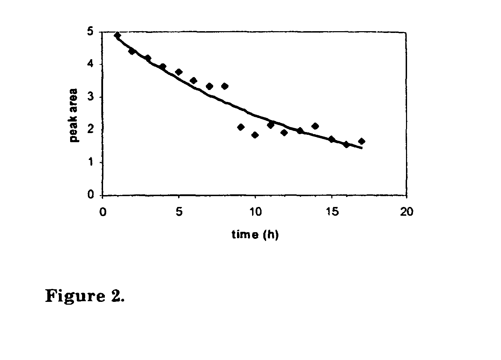

To check whether the polymer made in Example 2 in water had the same degradation rate as the monomer, degradation in D₂O buffered with Na₂HPO₄/NaH₂PO₄ at pH 7.4 was followed via ¹H-NMR. Degradation was followed by the integral of the peak at 4.8 ppm from HPMA-DMAE, which shifts when the alcohol is removed by hydrolysis. Figure 2 displays the peak integral of this proton as function of the time. As the degradation was followed for 19 hours and the half-life could be estimated from this curve, to be 12 hours. This estimated half-life corresponds to the half-life of the monomer at pH 7.4, thus indicating that the degradation rate of the polymer is the same as the monomer.

### Example 4: Biophysical characterisation of p(HPMA-DMAE) based polyplexes.

The DNA binding and condensation properties of p(HPMA-DMAE) were investigated by dynamic light scattering (DLS) and zeta potential measurements as a function of the polymer to DNA ratio.

More in detail, these physical characteristics of p(HPMA-DMAE) based polyplexes were investigated as a function of the polymer-nitrogen to DNA-phosphate (N/P) ratio. Plasmid DNA, plasmid pCMVLacZ, containing a bacterial LacZ gene preceded by a nuclear localization signal under control of a CMV promoter purchased from Sanvertech (Heerhugowaard, The Netherlands), was diluted in 20 mM HEPES buffer, pH 7.4, to a concentration of 75 µg/l. A stock solution of the polymer (5mg/ml) was diluted in the same buffer to various concentrations (7-450 µg/ml). Polyplexes were made by the addition 700 µl of a polymer solution to 175 µl DNA solution, mixed thoroughly and incubated for 30 minutes at room temperature. The Z-average diameters of the formed polyplexes were determined via dynamic light scattering (DLS) at 25°C and zeta-potential measurements were carried out as described by Van de Wetering *et al*. in Bioconjugate Chem. 10 (1999), 589-597. Z-average diameters of polyplexes were determined by dynamic light scattering (DLS) at 25 °C with a Malvern 4700 system using an argon-ion laser (488 nm) operating at 10.4 mW (Uniphase) and PCS (photon correlation spectrometry) software for Windows version 1.34 (Malvern, UK). For data analysis, the viscosity and refractive index of water was used. The system was calibrated with a polystyrene dispersion containing particles of 100 nm. For zeta potential, measurements were done at 25 °C in an aqueous DTS 5001cell with a Zetasizer 2000 unit (Malvern, UK) and PCS software version 1.34 (Malvern UK). For instrument calibration, a polystyrene dispersion with known zeta potential was used.

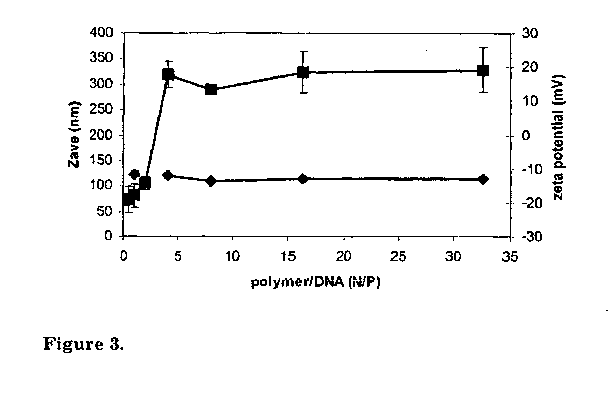

Figure 3 shows that when an excess of the polymer was added, the polymer was able to condense DNA into small particles with a size of approx. 110 nm bearing a positive zeta potential of approx. +19 mV. This is comparable to polyplexes composed of DNA and other cationic polymers such as pDMAEMA, pDAMA (polydiaminomethacrylate and particularly poly-(2-methacrylic acid 2-[(2-dimethyl)aminoethyl)-methylamino]-ethylester) and polyphosphazenes (prepared according to example 1 of WO-A-97/07226).

### Example 5: Destabilisation of polyplexes of p(HPMA-DMAE) and DNA.

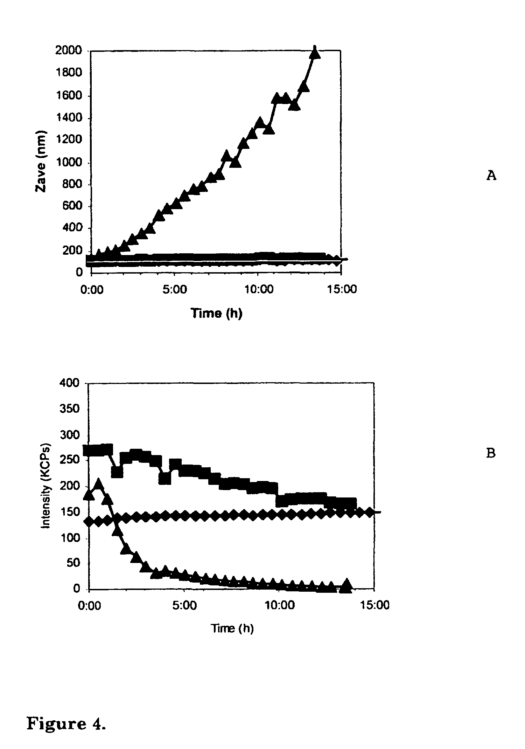

The destabilisation of p(HPMA-DMAE) based polyplexes (prepared as described in Example 4 (*vide infra*)) was investigated at pH 5.0 and 7.4 at 37°C by dynamic light scattering. Figure 4 depicts the particle size of the polyplexes (A) and the intensity of the signal (B) as function of time.

While the particle size of polyplexes based on p(HPMA-DMAE) at pH 5.0 or pDMAEMA at pH 7.4 stayed (almost) the same over the investigated time period, the particle size of p(HPMA-DMAE) based polyplexes at pH 7.4 increased dramatically in time. This indicates that the p(HPMA-DMAE) indeed degrades, and due to the decreased interaction between polymer and DNA, the particles will become larger. In time, the signal intensity also decreased at pH 7.4 for p(HPMA-DMEA) while remaining (almost) the same at pH 5.0 and for pDMAEMA (figure 4B), again indicating degradation of the p(HPMA-DMAE) at pH 7.4 and not at pH 5.0.

### Example 6: DNA release of polyplexes by degradation.

To investigate whether the DNA released by degradation from p(HPMA-DMAE) based polyplexes is still in its intact form, polyplexes, prepared according to Example 4, were incubated at 37 °C for different time points and subsequently subjected to gel electrophoresis.

Particularly, polyplexes of p(HPMA-DMAE) and plasmid DNA were made by mixing 10 µl plasmid solution, 200 µg/ml with 40 µl pHPMA-DMAE solution, 200 µg/ml, corresponding with a N/P ratio of 5.4 and incubated for 30 minutes at room temperature. This was done in 10 mM HEPES pH 7.4 or 10 mM sodium acetate pH 5, ionic strength adjusted to 150 mM with NaCl. As a control, pDMAEMA based polyplexes at pH 7.4 were also investigated. These polyplex dispersions were subsequently incubated at 37 °C for 0, 2, 4, 7, 24 or 48 hours. After cooling down to room temperature, 10 µl of the corresponding buffer and 3 µl sample buffer (0.4% (w/v) bromophenol blue, 10 mM EDTA, 50% (v/v) glycerol in water) were added to 20 µl of each polyplex dispersion. Thirty µl of these solutions were applied to a 0.7% agarose gel containing 0.5 µg/ml ethidium bromide and run at 100 V.

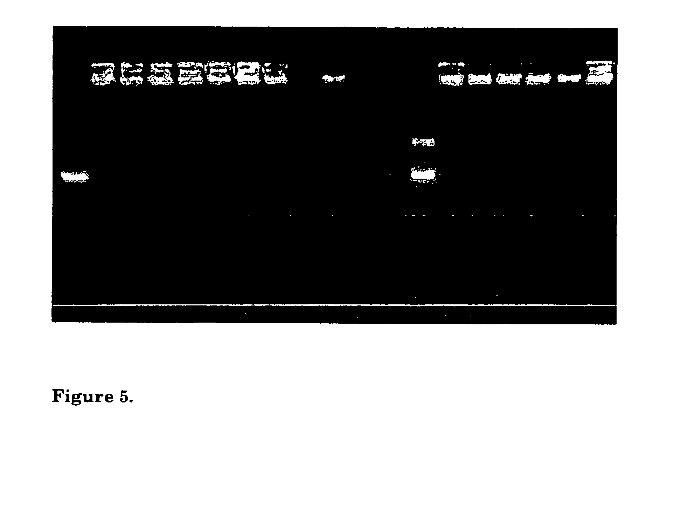

The sample of p(HPMA-DMEA) based polyplexes incubated for 2 hours at pH 7.4 was apparently lost during processing. Figure 5 shows the electrophoretic patterns of the polyplexes. For pDMAEMA at pH 7.4 or p(HPMA-DMAE) at pH 5.0, all DNA remained in the starting slot indicating that all DNA was still complexed with the polymer. At pH 7.4 and 48h incubation, all DNA was released from the p(HPMA-DMAE) based polyplexes, indicating that the polymer was degraded to such an extend, that complexation did not occur anymore. At 24h and 7h of incubation, no DNA could be detected anymore. This was also found for polyplexes when a polymer to DNA ratio N/P = 3 was chosen where large aggregates are formed, probably because ethidium bromide is not able to access the DNA anymore. Thus probably at these time points, the p(HPMA-DMAE) was partly degraded and because of decreased interaction between DNA and polymer, large aggregates were formed. This phenomenon is also described for pEI-DNA polyplexes in Bieber *et al*., J. Control. Release 82 (2002), 441-454.

### Example 7: Transfection efficiency and toxicity of p(HPMA-DMAE) based polyplexes.

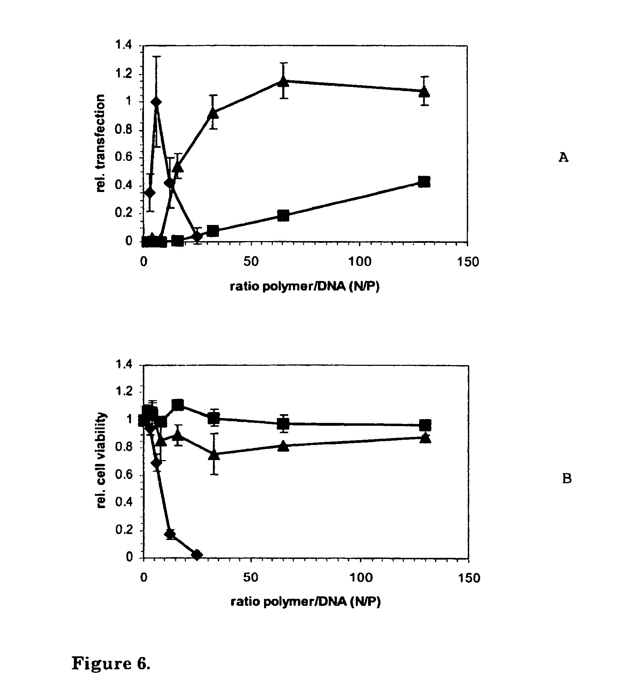

Figure 6 shows the transfection efficiency and toxicity of p(HPMA-DMAE) based polyplexes in CDS-7 cells in the absence or presence of the membrane disrupting peptide INF-7 as a function of the polymer to DNA ratio.

More in detail, transfection studies were done in COS-7 cells using the plasmid pCMVLacZ as reporter gene as described. In brief, 96 well plates were seeded with cells at a density of 3 x 104/cm, 24 h before transfection. At the day of transfection, polyplexes were prepared as follows: 150 µl polymer solution (various concentrations) was added to 50 µl plasmid solution (50 µg/ml), and after incubation for 30 minutes at room temperature, 50 µl of either buffer or INF-7 solution (150 µg/ml) was added and the polyplexes were incubated for another 15 minutes before addition to the cells. After rinsing the cells with HBS, 100 µl polyplex dispersion and 100 µl culture (with or without 10% serum) medium were incubated with the cells for 1 hour. After removal of the polyplex medium, fresh culture medium was added and the cells were incubated for another 48 hour. All transfection experiments were performed in two identical series in separate 96 well plates. One series was tested for reporter gene expression (β-galactosidase) by ONPG colorimetric assay, the other series was used to determine the number of viable cells using a XTT colorimetric assay. As a reference, pDMAEMA polyplexes prepared at the same DNA concentration and a N/P ratio of 6 were used. The transfection activity of this polyplex formulation was set at 1.

p(HPMA-DMAE) is not toxic to cells at all tested concentrations and is able to transfect cells, to some extend, especially at rather high polymer to DNA ratios. Low transfection efficiencies may be due to the inability of the polyplexes to escape from the endosome.

To increase endosomal escape, membrane disruptive peptides can be used. When the membrane destabilising peptide INF-7 was used in a transfection experiment with p(HPMA-DMAE) based polyplexes, an increase in the transfection efficiency was observed. At the same time some toxicity was observed, but this toxicity is independent of the polymer to DNA ratio, and thus probably caused by the peptide.

## Claims

1. A polymer based on a monomer of formula (I)
CH₂=C(R¹)-C(O)NH-CH₂-CHCH₃-O-C(O)-O-CH₂-CH₂-N(CH₃)₂ (I)
wherein R¹ is either a hydrogen atom, or a C₁₋₆-alkyl group.

2. The polymer of claim 1, being a homopolymer.

3. The polymer of claim 1 or 2, wherein R¹ is either a hydrogen atom or a C₁₋₃ alkyl group.

4. The polymer of claim 3, wherein R¹ is a methyl group.

5. Compound of the formula (I)
CH₂=C(R¹)-C(O)NH-CH₂-CHCH₃-O-C(O)-O-CH₂-CH₂-N(CH₃)₂ (I)
wherein R¹ is either a hydrogen atom, or a C₁₋₆-alkyl group.

6. The compound of claim 5, wherein R¹ is either a hydrogen atom or a C₁₋₃ alkyl group.

7. The compound of claim 6, wherein R¹ is a methyl group.

8. A method for preparing the compound of any one of claims 5-7, comprising the activation of N',N'-dimethylaminoethanol by 1,1'-carbonyldiimidazole; which activated alcohol is in a subsequent step (ii) reacted with N-(2-hydroxypropyl)-(R¹-substituted) acrylamide.

9. Synthetic transfection system comprising at least one cationic, water soluble or water dispersible polymer of any one of claims 1-4 as a carrier, and further comprising a nucleic acid fragment.

## Patentansprüche

1. Polymer, basierend auf einem Monomer der Formel (I)
CH₂=C(R¹)-C(O)NH-CH₂-CHCH₃-O-C(O)-O-CH₂-CH₂-N(CH₃)₂ (I)
worin R¹ entweder ein Wasserstoffatom oder eine C₁₋₆-Alkylgruppe darstellt.

2. Polymer nach Anspruch 1, bei dem es sich ein Homopolymer handelt.

3. Polymer nach Anspruch 1 oder 2, worin R¹ entweder ein Wasserstoffatom oder eine C₁₋₃₋Alkylgruppe darstellt.

4. Polymer nach Anspruch 3, worin R¹ eine Methylgruppe ist.

5. Verbindung der Formel (I)
CH₂=C(R¹)-C(O)NH-CH₂-CHCH₃-O-C(O)-O-CH₂-CH₂-N(CH₃)₂ (I)
worin R¹ entweder ein Wasserstoffatom oder eine C₁₋₆-Alkylgruppe darstellt.

6. Verbindung nach Anspruch 5, worin R¹ entweder ein Wasserstoffatom oder eine C₁₋₃₋Alkylgruppe darstellt.

7. Verbindung nach Anspruch 6, worin R¹ eine Methylgruppe ist.

8. Verfahren zur Herstellung der Verbindung nach einem der Ansprüche 5-7, umfassend die Aktivierung von N',N'-Dimethylaminoethanol mit 1,1 '-Carbonyldiimidazol, wobei der aktivierte Alkohol in einem folgenden Schritt (ii) mit N-(2-Hydroxypropyl)-(R¹⁻substituiertem)-Acrylamid umgesetzt wird.

9. Synthetisches Transfektionssystem, umfassend mindestens ein kationisches, wasserlösliches oder in Wasser dispergierbares Polymer nach einem der Ansprüche 1-4 als Träger und zusätzlich umfassend ein Nucleinsäurefragment.

## Revendications

1. Un polymère basé sur un monomère de formule (I):
CH2=C(R1)-C(O)NH-CH2-CHCH3-O-C(O)-O-CH2-CH2-N(CH3)2
dans laquelle R1 est soit un atome d'hydrogène, soit un radical C1-C6-alkyle.

2. Le polymère selon la revendication 1, consistant en un homopolymère.

3. Le polymère selon la revendication 1 ou 2, dans lequel R1 est soit un atome d'hydrogène, soit un radical C1-C3-alkyle.

4. Le polymère selon la revendication 3, dans lequel R1 est un radical méthyle.

5. Le composé de formule (I),
CH2=C(R1)-C(O)NH-CH2-CHCH3-O-C(O)-O-CH2-CH2-N(CH3)2
dans laquelle R1 est soit un atome d'hydrogène, soit un radical C1-C6-alkyle.

6. Le composé selon la revendication 5, dans lequel R1 est soit un atome d'hydrogène, soit un radical C1-C3-alkyle.

7. Le composé selon la revendication 6, dans lequel R1 est un radical méthyle.

8. Un procédé de préparation du composé selon une quelconque des revendications 5 à 7, comprenant l'activation de N',N'-diméthylaminoéthanol par le 1,1'-carbonyl-diimidasole, puis, dans une étape ultérieure (ii), la réaction de l'alcool activé résultant avec la N-(2-hydoxypropyl)-(R1-substitué)-acrylamide.

9. Un système de transfection synthétique comprenant au moins un polymère cationique, susceptible d'être dispersé ou solubilisée dans l'eau, selon une quelconque des revendications 1 à 4, en tant que support, et comprenant en outre un fragment d'acide nucléique.
